Europäisches Patentamt

(19) European Patent Office

Office européen des brevets

(11) Veröffentlichungsnummer: **0 004 567**
**B1**

(12) **EUROPÄISCHE PATENTSCHRIFT**

(45) Veröffentlichungstag der Patentschrift:
17.08.83

(51) Int. Cl.³: **A 61 B 3/02, A 61 B 3/04**

(21) Anmeldenummer: **79100688.5**

(22) Anmeldetag: **08.03.79**

(54) Vorrichtung zur subjektiven Augenrefraktionsbestimmung.

(30) Priorität: **07.04.78 DE 2815120**

(43) Veröffentlichungstag der Anmeldung:
**17.10.79 Patentblatt 79/21**

(45) Bekanntmachung des Hinweises auf die Patenterteilung:
**17.08.83 Patentblatt 83/33**

(84) Benannte Vertragsstaaten:
**DE FR GB IT**

(56) Entgegenhaltungen:
**DE-A-2 121 873**
**DE-B-1 225 892**
**US-A-3 969 020**

(73) Patentinhaber: **Herbert Schwind GmbH & Co. KG,**
**Goldbacher Strasse 57, D-8750 Aschaffenburg (DE)**

(72) Erfinder: *Reiner, Josef, Dr., Stefan-Lochner-Strasse 14,*
**D-5000 Köln 50 (DE)**

(74) Vertreter: **Grams, Klaus Dieter, Dipl.-Ing. et al,**
**Patentanwaltsbüro Tiedtke-Bühling-Kinne-**
**Grupe-Pellmann-Grams Bavariaring 4,**
**D-8000 München 2 (DE)**

EP 0 004 567 B1

Vorrichtung zur subjektiven Augenrefraktionsbestimmung

Die Erfindung bezieht sich auf eine Vorrichtung zur subjektiven Augenrefraktionsbestimmung gemäß dem Oberbegriff von Patentanspruch 1.

Bei der subjektiven Augenrefraktion mit Hilfe von Probiergläsern ist das erreichbare Ergebnis in hohem Maße von der Sehschärfe, der Beobachtungsgabe und der Kommunikationsfähigkeit des Probanden abhängig. Da der Proband während des Refraktionsvorganges entweder eine verhältnismäßig schwere Probierbrille aufsetzen und durch Probiergläser mit verhältnismäßig kleinem Durchmesser oder durch einen Phoropter mit Probiergläsern kleinen Durchmessers hindurchschauen muß, werden ihm diese Hilfsmittel im Laufe der Refraktionsbestimmung lästig und irritieren ihn wegen der Einengung des Gesichtsfeldes und eventueller Spiegelbilder infolge mehrerer hintereinandergeschalteter Probiergläser.

Durch die US-A-3 969 920 sind ein Verfahren und eine Vorrichtung zur subjektiven Augenrefraktionsbestimmung bekannt, bei dem bzw. der statt diskreter, austauschbarer Probiergläser ein Probierglassystem verwendet wird, dessen Brechkraft kontinuierlich veränderbar ist. Dieses Probierglassystem ist in einem Gehäuse unmittelbar vor den Augen des Probanden angeordnet, so daß der Proband in gleicher Weise gestört und irritiert wird, wie wenn die Probiergläser in einer Probierbrille oder einem Phoropter angeordnet sind, die sich unmittelbar vor seinen Augen befinden. Das bekannte Probierglassystem ist nach Art eines Teleskops aufgebaut und besteht im wesentlichen aus einer Objektivlinse, einem Okular und einer Spiegelanordnung, die die optische Weglänge zwischen dem Okular und der Objektivlinse verändern kann. Da dabei mehrere gegeneinander austauschbare Objektivlinsen unterschiedlicher Brechkraft vorgesehen sind, stellen diese Probiergläser dar und können die übrigen optischen Elemente des Probierglassystems als optisches System betrachtet werden.

Der Erfindung liegt die Aufgabe zugrunde, die gattungsgemäße Vorrichtung derart auszubilden, daß der Proband durch die Probiergläser bzw. das Probierglassystem weniger irritiert und abgelenkt wird.

Diese Aufgabe wird erfindungsgemäß dadurch gelöst, daß das optische System ein Bild des Probierglases in der Pupille des zu untersuchenden Auges oder in einem vorgewählten Abstand vor der Pupille erzeugt.

Da gemäß der Erfindung das jeweilige Probierglas zu dem zu untersuchenden Auge abgebildet wird, entfällt die Notwendigkeit, das Probierglas oder ein Probierglassystem selber in unmittelbarer Nähe des Auges anzuordnen. Obwohl somit das Probierglas in gewisser Entfernung vom Auge angeordnet ist, hat es aufgrund der Tatsache, daß es in die Pupille des zu untersuchenden Auges oder in einen vorgewählten Abstand vor der Pupille abgebildet wird, die gleiche Wirkung, wie wenn das Probierglas gegenständlich in einer vom Probanden getragenen Probierbrille oder in einem dicht vor dem Auge befindlichen Photopter angeordnet wäre.

In vorteilhafter Ausbildung der Erfindung kann vorgesehen sein, daß die Abbildung über einen Teilerspiegel erfolgt, über den das zu untersuchende Auge die Optotypen unter der Bedingung des Sehens im freien Raum wahrnehmen kann, wobei zu diesem Zweck vorzugsweise im bildseitigen Strahlengang des ersten optischen Abbildungselementes ein Teilerspiegel vorgesehen ist. Dies ermöglicht es, das Probierglas außerhalb des Blickfeldes des Probanden anzuordnen, so daß dem Probanden die Möglichkeit gegeben wird, in seiner Blickrichtung in den freien Raum zu sehen.

Weitere vorteilhafte Ausgestaltungen der Erfindung sind in den Unteransprüchen gekennzeichnet.

Ausführungsbeispiele der Erfindung sind in den Zeichnungen dargestellt und werden im folgenden näher erläutert. Es zeigt

Fig. 1 eine erfindungsgemäße Vorrichtung mit dem Strahlengang für einen Rechtsichtigen,

Fig. 2 die Vorrichtung gemäß Fig. 1 mit dem Strahlengang für einen Kurzsichtigen und

Fig. 3 eine gegenüber der in Fig. 1 gezeigten Darstellung abgewandelte Vorrichtung.

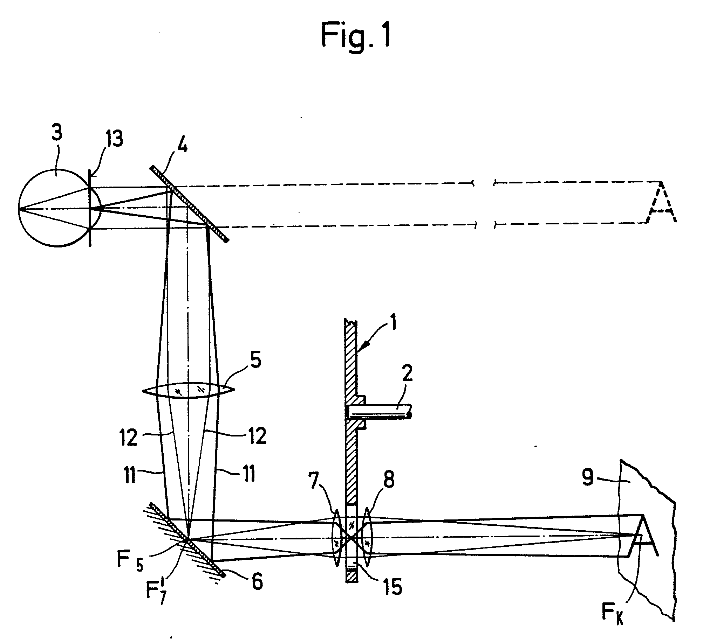

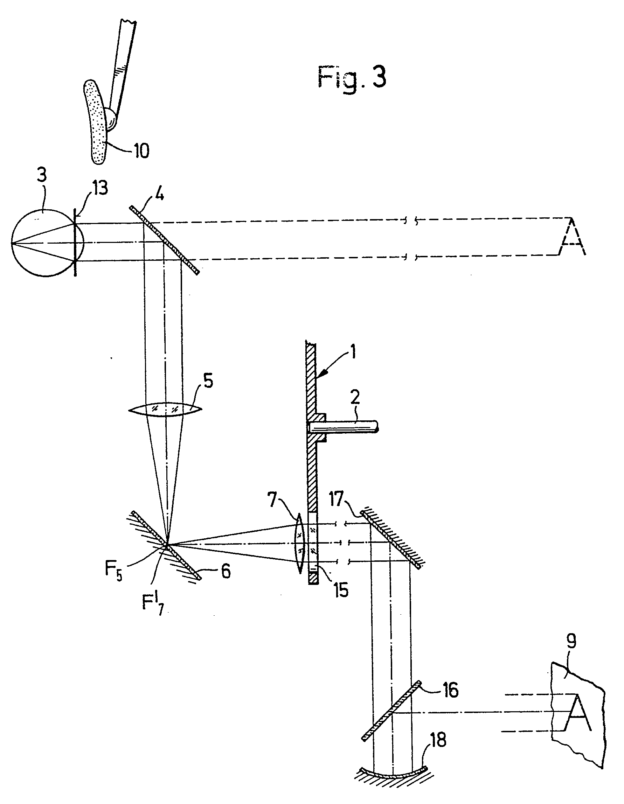

Die in Fig. 1 gezeigte Vorrichtung weist in dem Strahlengang von der dem zu untersuchenden Auge 3 zugewandten Eingangsseite her einen halbdurchlässigen Teilerspiegel 4, eine als erstes optisches Abbildungselement dienende Pluslinse 5, einen Spiegel 6 und einen um eine Achse 2 drehbaren Phoropter 1 auf. Der Phoropter weist in üblicher Weise eine Vielzahl verschiedenen starker Korrektionslinsen bzw. Probiergläser 15 auf, die in den Strahlengang einstellbar sind. In der gezeigten Einstellung ist die Korrektionswirkung des im Strahlengang befindlichen Probierglases 15 gleich Null. Hinter dem Phoropter ist ein als Linse ausgebildeter Kollimator 8 zur Abbildung der im Strahlengang nachfolgend angeordneten Optotypen vorgesehen. Vor dem Phoropter und so nahe wie möglich an diesem ist eine als zweites Abbildungselement dienende Pluslinse 7 im Strahlengang angeordnet, deren Brennweite gleich der zwischen Teilerspiegel 4 und Spiegel 6 angeordneten Pluslinse 5 ist und deren bildseitiger Brennpunkt $F'_7$ mit dem dingseitigen Brennpunkt $F_5$ zusammenfällt. Im Brennpunkt $F_K$ des Kollimators 8 ist eine gewünschtenfalls verkleinerte Optotypentafel 9 angeordnet. Die Linsen, Spiegel und die Optotypentafel sind mittels nicht dargestellter Fassungen in der Vorrichtung montiert. Die Vorrichtung weist ferner eine in Fig. 3 gezeigte Stirnstütze 10 auf, die so verstellbar mit dem Gehäuse verbunden ist, daß das zu untersuchende Auge des sich an die Stirnstütze anlehnenden Probanden einen vorbestimmten Scheitelabstand zu der Vorrichtung bzw. zu der Pluslinse 5 einnimmt.

In dem Strahlengang sind mittels der dicker gezeichneten Linien die Hauptstrahlen 11 und mittels der dünner gezeichneten Linien die Öffnungsstrahlen 12 gezeichnet.

Das sich annähernd in der doppelten Brennweite der Pluslinie 5 befindliche Probierglas 15 des Phoropters 1 wird in dem gezeigten Ausführungsbeispiel in die Hauptebene 13 des zu untersuchenden Auges 3 abgebildet, kann aber je nach Wunsch auch in einem dem Scheitelabstand eines Brillenglases entsprechenden Abstand vor dem Auge abgebildet werden: Damit wird die gleiche Wirkung erzielt, als würde der Proband durch das in einer Probierbrille oder in einem vor dem Auge befindlichen Phoropter befindliches Probierglas schauen. Der hinter dem Phoropter vorgesehene Kollimator 8 bildet die in seiner Brennebene angeordneten Optotypen über das Probierglas des Phoropters und die beiden Pluslinsen 5 und 7 im Unendlichen ab, so daß das Auge 3 die Optotypen über den Teilerspiegel 4 im freien Raum wahrnimmt.

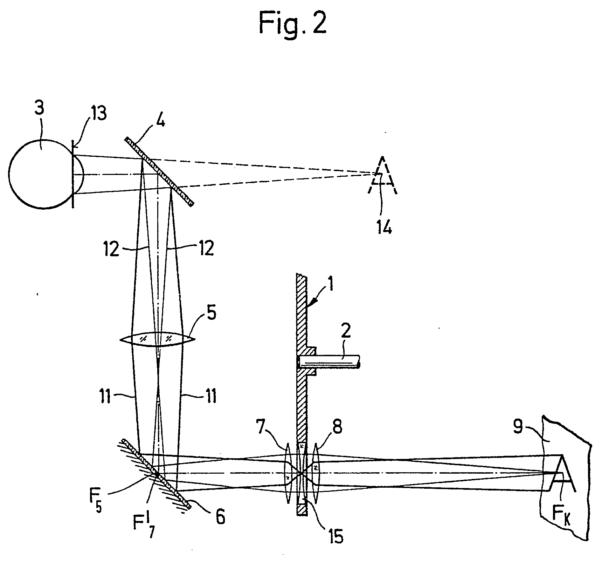

In Fig. 2 ist die in Fig. 1 gezeigte Vorrichtung gezeigt, mit der ein kurzsichtiges Auge 3 untersucht wird, dessen Fernpunkt 14 in einem Abstand vor dem Auge liegt. Das Probierglas 15 wird wiederum über die beiden Pluslinsen 5 und 7 in die Hauptebene 13 des zu untersuchenden Auges abgebildet. Durch die Bedingung, daß der dingseitige Brennpunkt $F_5$ mit dem bildseitigen Brennpunkt $F'_7$ der Linse 7 zusammenfällt und die Linse 5 einen Abstand annähernd gleich ihrer doppelten Brennweite vom Probierglas 15 des Phoropters aufweist, wird erreicht, daß die Optotype in der Ebene des Fernpunktes 14 des zu korrigierenden Auges abgebildet wird. Entsprechendes gilt für ein übersichtiges Auge.

Durch die Verwendung des Kollimators 8 wird erreicht, daß in geeigneter Weise verkleinerte Optotypentafeln wie Kleinbilddiapositive mit einer Vielzahl von Optotypen anstelle der bisher zu verwendenden großen Optotypentafeln verwendet werden können.

In Fig. 3 ist eine Ausführungsform gezeigt, die sich dadurch unterscheidet, daß anstelle des als Linse ausgebildeten Kollimators 8 ein als Hohlspiegel ausgebildeter Kollimator 18 verwendet wird, in dessen Brennebene sich wiederum die Optotypentafel 9 mit entsprechenden Optotypen befindet. Die Optotypentafel 9 wird über einen halbdurchlässigen Teilerspiegel 16 und einen Spiegel 17 in den oben beschriebenen Strahlengang aus Phoropter und den Pluslinsen 5, 7 sowie dem halbdurchlässigen Teilerspiegel 4 eingespiegelt. Mit dieser Vorrichtung wird in gleicher Weise erreicht, daß das Probierglas des Phoropters stets in einem gewünschten Abstand zum Auge abgebildet und die Optotypen im Fernpunkt des zu korrigierenden Auges abgebildet werden.

Für die binokulare Prüfung kann ein einheitliches Testzeichen verwendet werden, wenn man eine Kollimatorlinse oder einen Kollimatorspiegel verwendet, dessen Durchmesser größer ist als der Pupillenabstand. Bei Verwendung von zwei kleinen Linsen vor den Öffnungen des Phoropters müssen zwei gleichartige Testfiguren wie bei Stereoskopen verwendet werden.

Die Vorrichtung kann auch ohne den Kollimator 8 bzw. den Kollimator 18 verwendet werden, wenn die Optotypen in einem ausreichenden Abstand von beispielsweise 5 m, beispielsweise mittels einer Papptafel an der Wand, so dargeboten sind, daß der Proband diese durch das Probierglas 15 wahrnehmen kann.

In den oben beschriebenen Ausführungsbeispielen sind die Brennweiten der Pluslinsen 5 und 7 gleich, und die Hauptebene 13 des Auges 3 befindet sich in der doppelten Brennweite der Pluslinse 5. Die Probiergläser des Phoropters werden daher im Maßstab 1 : 1 in das Auge abgebildet. Die Probiergläser besitzen daher die gleiche Wirkung, wie wenn sie sich direkt vor dem Auge befinden würden. Benötigt ein Patient zur Korrektion beispielsweise −0,3 dpt, dann müssen auch die Probiergläser 15 im Phoropter −0,3 dpt betragen. Der Abbildungsmaßstab kann für die Probiergläser durch Ändern des Abstandes des Auges von der Linse 5 geändert werden. Beträgt er beispielsweise 1 : 2, dann ist auch der Dioptrinwert des Probierglases 15 entsprechend zu verändern. Auf diese Weise kann gewünschtenfalls das Probierglas 15 vergrößert abgebildet werden, so daß die Probiergläser einen sehr kleinen Durchmesser haben können und auf dem Phoropter damit eine große Vielzahl verschiedener Probiergläser anbringbar ist.

Die Vorrichtung ist von einem zur Klarheit der Figuren weggelassenen Gehäuse umgeben, das den Phoropter 1, die Pluslinsen 5 und 7 und den Spiegel 6 und gewünschtenfalls auch den Kollimator mit der Optotypentafel umschließt. Auf seiner Oberseite ist über der Pluslinie 5 eine Öffnung vorgesehen, über der der Teilerspiegel 4 angeordnet ist. Der Teilerspiegel 4 ist mittels einer geeigneten Halterung mit dem Gehäuse verbunden. Durch die Tatsache, daß der Teilerspiegel nicht innerhalb des Gehäuses angeordnet ist und der Proband somit nicht in das Gehäuse oder Gerät hineinsehen, sondern einfach durch den halbdurchlässigen Teilerspiegel hindurchschauen muß, wird der Eindruck des freien Sehens optimal erreicht.

In dem in Fig. 3 gezeigten Ausführungsbeispiel beträgt der Abstand zwischen den Optotypen und dem Kollimator 18 40 cm, zwischen dem Spiegel 17 und dem Phoropter ca. 20 cm, zwischen der Pluslinse 7 und der Pluslinse 5 ca. 30 cm und zwischen der Pluslinse 5 und dem Auge ebenfalls ca. 30 cm. Durch den relativ großen Abstand zwischen dem Spiegel 17 und dem Phoropter wird erreicht, daß weitere Meßelemente, beispielsweise Kreuzzylinder, in den Strahlengang von außen eingeführt werden können. Das Gehäuse kann zu diesem Zweck eine geeignete Öffnung aufweisen.

## Patentansprüche

1. Vorrichtung zur subjektiven Augenrefraktionsbestimmung, bei der das zu untersuchende Auge Optotypen sieht und die Sehschärfe vom Probanden beurteilt wird, mit zumindest einem Probierglas, das im Strahlengang von den Optotypen zum Auge angeordnet ist, und mit einem optischen System zwischen dem Probierglas und dem Auge, dadurch gekennzeichnet, daß das optische System (5, 7) ein Bild des Probierglases (15) in der Pupille des zu untersuchenden Auges (3) oder in einem vorgewählten Abstand vor der Pupille erzeugt.

2. Vorrichtung nach Anspruch 1, gekennzeichnet durch eine das Probierglas (15) zu dem zu untersuchenden Auge (3) hin abbildendes erstes optisches Abbildungselement (5).

3. Vorrichtung nach Anspruch 2, dadurch gekennzeichnet, daß augenseitig nahe am Probierglas (15) ein zweites optisches Abbildungselement (7) vorgesehen ist, dessen bildseitiger Brennpunkt (F'₇) mit dem dingseitigen Brennpunkt (F₅) des ersten optischen Abbildungselementes (5) zusammenfällt.

4. Vorrichtung nach Anspruch 3, dadurch gekennzeichnet, daß die Brennweite des ersten optischen Abbildungselementes (5) gleich der des zweiten optischen Abbildungselementes (7) ist.

5. Vorrichtung nach einem der Ansprüche 2 bis 4, dadurch gekennzeichnet, daß im bildseitigen Strahlengang des ersten optischen Abbildungselementes (5) ein Teilerspiegel (4) vorgesehen ist.

6. Vorrichtung nach einem der Ansprüche 3 bis 5, dadurch gekennzeichnet, daß das Probierglas (15) annähernd in der Ebene der doppelten Brennweite des ersten optischen Abbildungselementes (5) vorgesehen ist.

7. Vorrichtung nach einem der Ansprüche 3 bis 6, dadurch gekennzeichnet, daß im Strahlengang vor dem Probierglas (15) ein Kollimator (8, 18) vorgesehen ist, in dessen Brennebene die Optotypen angeordnet sind.

8. Vorrichtung nach Anspruch 7, dadurch gekennzeichnet, daß die Brennweite des Kollimators (8, 18) zur Einstellung der Vergrößerung der Optotypen veränderbar ist.

9. Vorrichtung nach einem der Ansprüche 1 bis 8, dadurch gekennzeichnet, daß eine Stirnstütze (10) vorgesehen ist, die zur Einstellung des Abstandes des Auges (3) zum Teilerspiegel (4) verschiebbar angeordnet ist.

## Claims

1. Device for the subjective determination of eye refraction, wherein the eye to be examined sees optotypes and the visual power or acuity of subjects is assessed, comprising at least one test lens, which is disposed in the optical path from the optotypes to the eye, and an optical system between the test lens and the eye, characterised in that the optical system (5, 7) forms an image of the test lens (15) in the pupil of the eye (3) to be examined or at a preselected distance in front of the pupil.

2. Device according to Claim 1, characterised by a first optical image-forming element (5) which projects an image of the test lens (15) towards the eye (3) to be examined.

3. Device according to Claim 2, characterised in that on the eye side, near to the test lens (15), there is provided a second optical image-forming element (7) having, on the image side, a focal point (F'₇) which coincides with, on the object side, the focal point (F₅) of the first optical image-forming element (5).

4. Device according to Claim 3, characterised in that the focal length of the first optical image-forming element (5) is equal to that of the second optical image-forming element (7).

5. Device according to any one of Claims 2 to 4, characterised in that a beam-splitting reflector (4) is provided in the optical path, on the image side, of the first optical image-forming element (5).

6. Device according to any one of Claims 3 to 5, characterised in that the test lens (15) is provided approximately in the plane which is twice the focal length of the first optical image-forming element (5).

7. Device according to any one of Claims 3 to 6, characterised in that there is provided, in the optical path in front of the test lens (15), a collimator (8, 18) in the focal plane of which the optotypes are arranged.

8. Device according to Claim 7, characterised in that the focal length of the collimator (8, 18) can be varied to adjust the magnification of the optotypes.

9. Device according to any one of Claims 1 5, characterised in that there is provided an end support (10) which is movably mounted to adjust the distance between the eye (3) and the beam-splitting mirror (4).

## Revendications

1. Dispositif pour la détermination subjective de la réfraction des yeux, dans lequel l'oeil à examiner voit des caractères optiques et où l'on apprécie l'acuite de vision du sujet, avec au moins un verre d'essai disposé sur le trajet des rayons lumineux allant de ces caractères à l'oeil et avec un système optique prévu entre ce verre et l'oeil, caractérisé en ce que le système optique (5, 7) réalise une image du verre d'essai (15) dans la pupille de l'oeil (3) à examiner ou à une distance prédéterminée en avant de celle-ci.

2. Dispositif suivant la revendication 1, caractérisé par un premier élément optique de réalisation d'image (5) qui réalise celle du verre d'essai (15) pour l'oeil à examiner (3).

3. Dispositif suivant la revendication 2, caractérisé en ce que près du verre d'essai (15) et du côté de l'oeil il est prévu un second élément de formation d'image (7) dont le foyer côté image (F'7) coincide avec celui côté objet (F5) du premier élément optique (5).

4. Dispositif suivant la revendication 3, caractérisé en ce que la distance focale du premier élément optique de formation d'image (5) est égale à celle du second (7).

5. Dispositif suivant l'une des revendications 2 à 4, caractérisé en ce que sur le trajet des rayons lumineux côté image du premier élément optique de formation d'une telle image (5) il est prévu un miroir semi-transparent (4).

6. Dispositif suivant l'une des revendications 3 à 5, caractérisé en ce que le verre d'essai (15) est prévu a peu près dans le plan correspondant au double de la distance focale du premier élément optique de formation d'image (5).

7. Dispositif suivant l'une des revendications 3 à 6, caractérisé en ce que sur le trajet des rayons lumineux en avant du verre d'essai (15) il est prévu un collimateur (8, 18) dans le plan focal duquel les caractères optiques sont disposés.

8. Dispositif suivant la revendication 7, caractérisé en ce que la distance focale du collimateur (8, 18) peut être modifiée en vue du réglage du grossissement des caractères optiques.

9. Dispositif suivant l'une des revendications 1 à 8, caractérisé en ce qu'il est prévu un appui frontal (20), lequel est disposé de façon à pouvoir être deplacé en vue du réglage de la distance entre l'oeil (3) et le miroir semi-transparent (4).

# Fig.1

# Fig. 2

# Fig. 3